# EUROPEAN PATENT SPECIFICATION

(11) **EP 2 807 146 B1**
(45) Date of publication and mention of the grant of the patent: **13.09.2017**
(21) Application number: 13741266.4
(22) Date of filing: 28.01.2013
(51) Int. Cl.: C07C 319/18, C07B 45/06, B01J 23/52, B01J 27/122, C07C 323/22

(54) **PREPARATION OF [ALPHA]-SULFENYLATED CARBONYL COMPOUNDS FROM PROPARGYLIC ALCOHOLS IN ONE STEP**
HERSTELLUNG VON [ALPHA]-SULFENYLIERTEN CARBONVERBINDUNGEN AUS PROPARGYLALKOHOLEN IN EINEM SCHRITT
PRÉPARATION DE COMPOSÉS [ALPHA]-SULFÉNYLÉS CARBONYLE À PARTIR D'ALCOOLS PROPARGYLIQUES EN UNE SEULE ÉTAPE

(30) Priority: 27.01.2012 SE 1250057
(43) Date of publication of application: 03.12.2014
(73) Proprietor: Kat2Biz AB, 114 36 Stockholm (SE)
(72) Inventor: SAMEC, Joseph, SE-163 67 Spånga (SE); BISWAS, Srijit, Lucknow - 226014, Uttar Pradesh (IN)
(74) Representative: Brann AB
(86) International application number: PCT/SE2013/050063
(87) International publication number: WO 2013/112104

(56) References cited:
- Y. ISHINO ET AL: "Novel synthesis of 4,5-bis (arylthio)-2,3,4,5-tetrahydro-1-benzothiep ins: Noteworthy cyclization by the reaction of 2-butynediol with arenethiols in the presence of zinc iodide", SYNTHESIS., vol. 9, 1987, pages 827-829, XP002743520, DEGEORG THIEME VERLAG, STUTTGART. ISSN: 0039-7881
- DZHAFAROV ET AL: "Direction of the nucleophilic addition of arylthiols to acetylenic alcohols", ZHURNAL ORGANICHESKOI KHIMII, MAIK NAUKA, MOSCOW, RU, vol. 18, no. 4, 1 January 1982 (1982-01-01), pages 739-743, XP008174558, ISSN: 0514-7492
- Y. ISHINO ET AL: "A novel synthesis of 3,4-dihydro-2H-1-benzothiopyrans. Acid-catalyzed intermolecular cycloaddition of acetylenice alcohols witharenethiols.", CHEMISTRY LETTERS, vol. 7, 1990, pages 1185-1188, XP002743521, Chemical society of Japan ISSN: 0366-7022
- SRIJIT BISWAS ET AL: "A gold(i)-catalyzed route to [alpha]-sulfenylated carbonyl compounds from propargylic alcohols and aryl thiols", CHEMICAL COMMUNICATIONS, vol. 48, no. 52, 1 January 2012 (2012-01-01), pages 6586-6588, XP055081977, ISSN: 1359-7345, DOI: 10.1039/c2cc32042h
- BISWAS S. ET AL: 'A gold(I)-catalyzed route to [alpha]-sulfenylated carbonyl compounds from propargylic alcohols and aryl thiols' CHEMICAL COMMUNICATIONS vol. 48, no. 52, 04 July 2012, pages 6586 - 6588, XP055081977
- XIAO, WEN-JING ET AL.: 'Highly stereoselective palladium- catalyzed dithiocarbonylation of propargylic mesylates with thiols and carbon monoxide' JOURNAL OF THE AMERICAN CHEMICAL SOCIETY vol. 70, 2005, pages 1802 - 1807, XP055081978
- TROST B. M.: '[alpha]-Sulfenylated carbonyl compounds in organic synthesis' CHEMICAL REVIEWS vol. 78, no. 4, 1978, pages 363 - 382, XP055081981
- DZHAFAROV, A. A. ET AL.: 'Direction of the nucleophilic addition of arylthiols to acetylenic alcohols' ZHURNAL ORGANICHESKOI KHIMII vol. 18, no. 4, 1982, pages 739 - 743, XP008174558
- GEORGY, M. ET AL.: 'Gold(III)-catalyzed direct nucleophilic substitution of propargylic alcohols' TETRAHEDRON vol. 65, 2009, pages 1758 - 1766, XP025908855
- DAVIES, P.W. ET AL.: 'Alkynes as masked ylides: Gold-catalysed intermolecular reactions of propargylic carboxylates with sulfides' CHEMICAL COMMUNICATIONS 2008, ISSN 1359-7345 pages 238 - 240, XP055081983
- DAVIES, P.W. ET AL.: 'A reactivity switch in the gold-catalysed coupling of allyl sulfides with propargylic carboxylates' SYNLETT vol. 23, 2012, pages 70 - 73, XP055081986

## Description

### TECHNICAL FIELD

The present invention relates to an atom efficient methodology to prepare α-sulfenylated carbonyl compounds from propargylic alcohols and thiols by means of transition metal catalysis

### BACKGROUND

Alfa sulfenylated carbonyl compounds are a group of compounds many of which are the active substances in pharmaceutical compositions. The problem with these compounds is the synthetic procedure which could both involve toxic substances and multiple steps. Synthetic procedures attempting to avoid these drawbacks usually fail to present high yields of the wanted product or produce a variety of by-products.

The traditional synthesis of α-sulfenylated carbonyl compounds involves substitution of the corresponding α-halogenated intermediate by sulphide anions. These toxic and difficult to handle α-halogenated carbonyl intermediates are synthesized in advance and the synthesis associated with the generation of stoichiometric amounts of chemical waste both in the preparation of the α-halogenated carbonyl compounds and in the subsequent substitution reaction to generate the α-sulfenylated carbonyl product.

Alternatively, the α-sulfenylated carbonyl compound can be synthesized by the reaction of a parent carbonyl compound or preformed enolate with sulfenylating agents such as disulfides, *N*-(phenylsulfanyl)succinimides, or sulfenyl chlorides). However, these reactions require an additional synthetic step in the preparation of the sulfenylating agent and also lead to the formation of a stoichiometric amount of waste in the carbon sulphur bond forming reaction. Asymmetric versions of such stoichiometric electrophilic *α*-sulfenylations have been reported using a chiral auxiliary, an organocatalyst or chiral complexes of titanium(IV) or nickel(II).
It is known that transition metals catalyze a number of chemical reactions. For example, Weng-Jing Xiao et al., J Organic Chemistry, 2005, 70, 1802-1807, uses palladium for carbonylation of propargylic mesylates using thiols and carbon monoxide to produce thioesters.

Yoshio Ishino et al. disclose in Communications 827-829 (1987) a zinc catalysed process which yields a minor amount of alpha-sulfenylated carbonyl compound starting from 2-butynediols.

The present invention solves the problem with the traditional synthesis and the synthetic procedures suggested by prior art.

### SUMMARY OF THE INVENTION

The object of the present invention is to provide a method to generate α-sulfenylated carbonyl compounds starting from a primary or secondary propargylic alcohol and a thiol with up to 100% atom efficiency. To the knowledge of the present inventors this has never before been presented.

One aspect of the present invention relates to a method of preparing an α-sulfenylated carbonyl compound comprising providing reactants comprising a primary or a secondary propargylic alcohol, a thiol, a transition metal catalyst wherein the catalyst is a catalyst comprising gold(III), gold(I), copper(I), palladium(II), platinum(II) or mixtures thereof such as gold and palladium, or copper and palladium, or platinum and palladium and a solvent and allowing the reactants to react.

Preferred embodiments of the above mentioned aspects are described below, all the embodiments below should be understood to refer to the aspects described above.

In one embodiment the method is performed in one step.

In one embodiment the propargylic alcohol is a primary alcohol.

In another embodiment the propargylic alcohol is a secondary alcohol.

In another embodiment the solvent is selected from dichloroethane, chloroform, carbon tetrachloride, water, or dichloromethane or mixtures thereof.

In another embodiment the reaction is conducted at a temperature of at least 25°C, preferably 50-100 °C.

In another embodiment the thiol is an aryl thiol.

In another embodiment the thiol is an alkyl thiol.

In another embodiment the compound is a benzenethiol.

In another embodiment the phenyl group of the propargylic alcohol is substituted in either ortho, meta or para position.

In another embodiment the reaction is conducted using a catalyst comprising gold(I) or gold (III).

In another embodiment the reaction is conducted using a catalyst comprising copper(I).

In another embodiment the reaction is conducted using a catalyst comprising platinum(II).

In another embodiment the reaction is conducted using a catalyst comprising gold(I) chloride or gold(III) chloride.

In another embodiment the reaction is conducted using a catalyst comprising copper(I) chloride, copper(I) bromide, copper(I) iodide, copper(I) trifluoroacetate, gold(I)chloride, gold(I)bromide or gold(I)iodide.

In another embodiment the reaction is conducted using a catalyst comprising platinum(II) chloride.

In another embodiment the reaction is conducted using a catalyst comprising gold(III) chloride or gold(I) chloride together with an aryl thiol.

In another embodiment the reaction is conducted using a catalyst comprising copper(I) chloride, copper(I) bromide, copper(I) iodide or copper(I) trifluoroacetate together with an aryl thiol.

In another embodiment the reaction is conducted using a compound generated from gold(III) chloride or gold(I) chloride together with an aryl thiol.

In another embodiment the reaction is conducted using a compound generated from copper(I) chloride, copper(I) bromide, copper(I) iodide or copper(I) trifluoroacetate together with an aryl thiol.

In another embodiment the reaction is conducted using a compound generated from platinum(II) chloride together with an aryl thiol.

The present invention could be used to transform alcohols to yield the corresponding α-sulfenylated carbonyl compounds in good to excellent yield, i.e. 55 to 100% yield.

In another embodiment the catalyst is used in 0.1-10 mol%, for example 0.1 mol% or more, or 0.3 mol% or more, or 0.5 mol% or more, or 0.7 mol% or more, or 1 mol% or more, or 3 mol% or more, or 5 mol% or more, or 7 mol% or more, or 10 mol% or less.

A second aspect of the present invention relates to the use of a primary or a secondary propargylic alcohol and a thiol, a transition metal catalyst wherein the catalyst is a catalyst comprising gold(III), gold(I), copper(I), palladium(II), platinum(II) or mixtures thereof such as gold and palladium, or copper and palladium, or platinum and palladium and a solvent for preparing an α-sulfenylated carbonyl compound.

The preferred embodiments of the second aspect are the same as for the first embodiment.

### DETAILED DESCRIPTION

The invention relates to a method where primary and secondary propargylic alcohols are converted into the corresponding α-sulfenylated carbonyl compounds.

Scheme 1 discloses a schematic scheme of the traditional synthetic procedure to prepare α-sulfenylated carbonyl compounds consisting of two step procedures here scheme 1 only disclose the last step. These procedures produce chemical waste as side products, M is a metal, X is a halide, and Y is a leaving group.

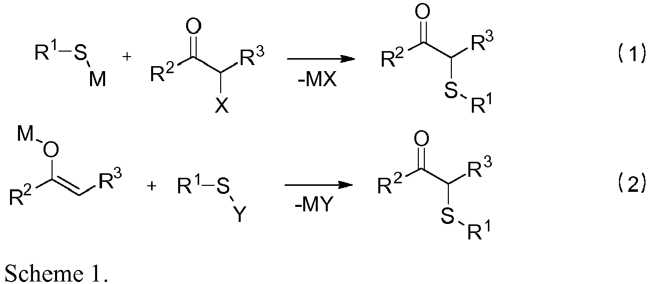

The present invention makes the formation of sulfenylated carbonyl compounds easier since preferably the present invention is a one step synthesis where all reactants are added to a flask or a reaction container and no intermediate steps or isolation steps are required to obtain the final product. However, the final product is preferably purified to remove unreacted substances or solvent for example. The reactants, that is the alcohol, thiol and catalyst could also be added continuously or drop wise to the reaction. The reactants and solvent can be mixed by stirring or shaking or any other suitable method.

The present inventors found that by starting with propargylic alcohol, which is commercially available or could be synthesized fairly easy, the α-sulfenylated carbonyl compound is obtainable by simply adding a thiol, an aryl thiol or an alkyl thiol, in the presence of a transition metal catalyst, Scheme 2.

Referring to scheme 2, the alcohol is a primary or secondary alcohol. In other words R₂ could be hydrogen, but could also be an alkyl group. R₁ could be a hydrogen, an alkyl, or an aryl group.

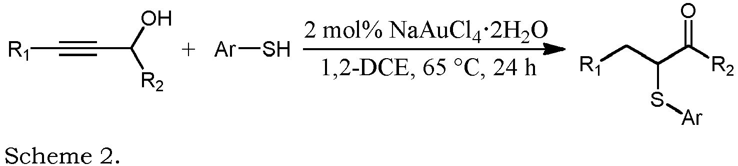

As aryl thiol, any aryl thiol could be used for example benzene thiol or substituted benzene thiol for example halogenated benzene thiol or alkyl substituted aryl benzene thiol. The aryl group, in R₂ or in the thiol, may be substituted in ortho, meta or para position.

As alkyl thiol, any alkyl group could be used. For example the alkyl group could be a straight chain of hydrocarbons or it could be branched or substituted. The alkyl group could be a methyl, ethyl, propyl, butyl, pentyl, hexyl or longer and any conformational isomer. The alkyl could also have a ring structure such as cyclo pentyl or cyclo hexyl.

The method of preparing an *α*-sulfenylated carbonyl compound according to the present invention comprises:
- providing reactants comprising a primary or a secondary propargylic alcohol, a thiol and a transition metal catalyst;
- providing a solvent;
- mixing the reactants and the solvent;
- letting the reactants react.

The reaction is performed using transition metal catalysts (e.g. gold, palladium, platinum, copper and so on), preferably late transition metals catalysts (gold, palladium, platinum), to generate the α-sulfenylated carbonyl compounds in good to excellent yields (55-100% yield) with no or very little by-products, the remaining is starting reactants. The transition metal catalyst may comprise one or more transition metals or is a mixture of one or more transition metal catalysts. The only observed by-product is thioether in small amounts. The total amount of catalyst should be between 0.1 and 10 mol%, preferably between 1 and 5 mol% for example 1 mol% or more, or 2 mol% or more, or 3 mol% or 5 mol% or less, or 4 mol% or less. In a preferred embodiment, a gold(I) catalyst is used in an amount of 1 to 5 mol%, and in another embodiment a gold(III) catalyst is used in an amount of 1 to 5 mol%. The gold(I) or gold(III) comprising catalyst could also be reacted with an aryl thiol to generate a compound to be used in the synthesis of the α-sulfenylated carbonyl compound. Preferred gold catalysts comprises gold(I) chloride or gold(III) chloride catalyst and could be for example NaAuCl₄·2H₂O. In another preferred embodiment, a copper(I) catalyst is used in an amount of 1 to 5 mol%. In yet another embodiment a gold(I) chloride catalyst is used together with benzene thiol. As mentioned above the transition metal catalyst may comprise a mixture of transition metals for example gold(I) and palladium(II) and in one embodiment this mixture is used together with an alkylthiol.

The reaction can be performed under mild reaction conditions (25°C - 100 °C, for example 25°C or more, or 35°C or more, or 55 °C or more or 80°C or less, or 75°C or less, or 65°C or less) by conventional heating or by heating in a microwave oven. In a typical experiment the reaction is conducted between 50 and 100°C. The reaction can be conducted in a solvent selected from but not limited to water, dichloromethane, dichloroethane, chloroform, carbon tetrachloride or any other polar solvent or mixtures thereof. Typically the reaction is performed during 10-30 hours such as 24 hours but could continue for 48 hours or a week.

In order to inhibit disulfide formation and other side reactions, the reaction should preferably be conducted in an inert atmosphere. The inert atmosphere may also comprise nitrogen or argon and the reaction could be conducted in a glove box comprising one of said gases. However, the method of the present invention can also be performed in an open atmosphere.

The present invention can for example be used to produce phenyl(4-phenylbut-3-yn-2-yl)sulfane, 1-phenyl-2-(phenylthio)hexane-3-one, 4-methyl-1-phenyl-2-(phenylthio)pentane-3-one, 3-(phenylthio)hexane-2-one, phenyl(1,3-diphenylprop-2-ynyl)sulfane, 3-phenyl-2-(phenylthio)propanal, 3-(naphthalene-4-yl)-2-(phenylthio)propanal, 3-(naphthalene-4-yl)-2-(phenylthio)propanal, 2-(4-bromophenylthio)-3-phenylpropanal, 2-(4-chlorophenylthio)-3-phenylpropanal, 2-(4-fluorophenylthio)-3-phenylpropanal, 2-(4-isopropylphenylthio)-3-phenylpropanal and 2-(4-methoxyphenylthio)-3-phenylpropanal.

The present invention also relates to a kit for preparing an *α*-sulfenylated carbonyl compound comprising a primary or a secondary propargylic alcohol and a thiol, a transition metal catalyst and a solvent. The thiol could be an aryl thiol or an alkyl thiol. Said components could be arranged in one or more containers, preferably two or three containers and the contents of the containers are then mixed in order to initiate the reaction. The kit should contain at least one transition metal catalyst or a mixture of transition metal catalysts.

### EXAMPLES

### Example 1. Preparation of 4-Phenyl-3-(phenylthio)butane-2-one.

At first, the catalyst NaAuCl₄·2H₂O (8 mg, 2 mol%) was weighed and transferred to a 5 mL microwave vial containing a small magnet in a glove box under nitrogen atmosphere. After that, the cap of the vial was closed tightly and the vial was taken out from the glove-box. 2.5 mL of dry 1,2-dichloroethane solvent followed by 4-phenyl-3-butyn-2-ol (145 µL, 1 mmol) and benzenethiol (154 µL, 1.5 mmol) were added to the vial by syringe and was stirred using a magnetic stirrer at 65 °C for 24 h. After completion of the reaction (by TLC), 1,2 dichloroethane was evaporated under reduced pressure and the residue was purified by silica-gel (100-200 mess) column chromatography using 3% (*v*/*v*) ethyl acetate / pentane solution to afford the desired product as a pale yellow oil (233 mg, 0.91 mmol, 91% yield). ¹H NMR (300 MHz, CDCl₃): *δ* = 2.20 (s, 3 H, H-1), 3.00 (dd, *J =* 6.9 Hz, 14.4 Hz, 1 H, H-4), 2.68 (dd, *J =* 8.4 Hz, 14.1 Hz, 1 H, H-4), 3.90 (dd, *J* = 6.9 Hz, 8.4 Hz, 1 H, H-3), 7.18-7.37 (m, 10 H, H-arom) ppm. ¹³C NMR (75 MHz, CDCl₃): *δ* = 28.1, 36.9, 59.0, 127.1, 128.5, 128.8, 129.4, 133.0, 133.3, 138.3, 204.5 ppm.

### Example 2. Preparation of 1-Phenyl-2-(phenylthio)hexane-3-one.

1-phenylhex-1-yn-3-ol (184 µL, 1 mmol), benzenethiol (154 µL, 1.5 mmol) and the catalyst NaAuCl₄·2H₂O (8 mg, 2 mol%) in 1,2-dichloroethane solvent (2.5 mL) were treated as described for 4-Phenyl-3-(phenylthio)butane-2-one to obtain the product as a yellowish oil (256 mg, 0.90 mmol, 90% yield). IR (Neat): *v* = 2962, 1706, 1439, 690 cm⁻¹. ¹H NMR (300 MHz, CDCl₃): *δ* = 0.80 (t, *J =* 7.2 Hz, 3 H, H-6), 1.43-1.54 (m, 2 H, H-5), 2.29-2.40 (m, 1 H, H-4), 2.47-2.58 (m, 1 H, H-4), 2.98 (dd, *J* = 6.3, 14.1 Hz, 1 H, H-1), 3.19 (dd, *J =* 8.7, 14.1 Hz, 1 H, H-1), 3.88 (dd, *J* = 6.6, 8.7 Hz, 1 H, H-2), 7.16-7.37 (m, 10 H, H-arom) ppm. ¹³C NMR (100 MHz, CDCl₃): *δ* = 13.5, 17.2, 37.0, 42.8, 58.0, 126.6, 128.0, 128.4, 129.0, 129.1, 133.0, 133.1, 138.3, 206.0 ppm. HRMS: calcd. for C₁₈H₂₀OSNa 307.1133; found 307.1115.

### Example 3. Preparation of 4-Methyl-1-phenyl-2-(phenylthio)pentane-3-one.

4-methyl-1-phenylpent-1-yn-3-ol (128 µL, 1 mmol), benzenethiol (154 µL, 1.5 mmol) and the catalyst NaAuCl₄·2H₂O (8 mg, 2 mol%) in 1,2-dichloroethane solvent (2.5 mL) were treated as described for 4-Phenyl-3-(phenylthio)butane-2-one to obtain the product as a yellowish oil (245 mg, 0.86 mmol, 86% yield). IR (Neat): *v* = 2969, 1707, 1439, 699 cm⁻¹. ¹H NMR (300 MHz, CDCl₃): 6 = 0.76 (d, *J* = 6.9 Hz, 3 H, H-5), 1.04 (d, *J =* 6.7 Hz, 3 H, CH₃), 2.70-2.79 (m, 1 H, H-4), 3.00 (dd, *J* = 5.7 Hz, 13.8 Hz, 1 H, H-1), 3.22 (dd, *J =* 9.6 Hz, 13.8 Hz, 1 H, H-1), 3.97 22 (dd, *J* = 5.7 Hz, 9.6 Hz, 1 H, H-2), 7.42-7.38 (m, 2 H, H-arom), 7.14-7.35 (m, 8 H, H-arom) ppm. ¹³C NMR (100 MHz, CDCl₃): *δ* = 17.8, 18.3, 37.3, 39.3, 56.5, 126.5, 128.3, 129.0, 129.2, 132.8, 133.6, 138.5, 208.7 ppm. HRMS: calcd. for C₁₈H₂₀OSNa 307.1133; found 307.1115.

### Example 4. Preparation of 3-(Phenylthio)hexane-2-one.

hex-3-yn-2-ol (324 µL, 3 mmol), benzenethiol (103 µL, 1 mmol) and the catalyst NaAuCl₄·2H₂O (8 mg, 2 mol%) in 1,2-dichloroethane solvent (2.5 mL) were treated as described for 4-Phenyl-3-(phenylthio)butane-2-one to obtain the product as a yellowish oil (105 mg, 0.50 mmol, 50% yield). ¹H NMR (300 MHz, CDCl₃): *δ* = 0.96 (t, *J =* 7.2 Hz, 3 H, H-6), 1.38-1.60 (m, 2 H, H-5), 1.64-1.86 (m, 2 H, H-4), 2.26 (s, 3 H, H-1), 3.65 (t, *J =* 7.5 Hz, 1 H, H-3), 7.24-7.44 (m, 5 H, H-arom) ppm. ¹³C NMR (75 MHz, CDCl₃): *δ* = 14.0, 20.8, 26.6, 32.7, 57.8, 128.1, 129.3, 132.4, 133.4, 205.8 ppm.

### Example 5. Preparation of 3-Phenyl-2-(phenylthio)propanal.

3-phenylprop-2-yn-1-ol (125 µL, 1 mmol), benzenethiol (154 µL, 1.5 mmol) and the catalyst NaAuCl₄·2H₂O (8 mg, 2 mol%) in 1,2-dichloroethane solvent (2.5 mL) were treated as described for 4-Phenyl-3-(phenylthio)butane-2-one to obtain the product as a reddish brown oil (216 mg, 0.89 mmol, 89% yield). ¹H NMR (300 MHz, CDCl₃): *δ* = 3.00 (dd, *J =* 6.9 Hz, 14.4 Hz, 1 H, H-3), 3.22 (dd, *J =* 8.1 Hz, 14.4 Hz, 1 H, H-3), 3.81-3.87 (m, 1 H, H-2), 7.23-7.41 (m, 10 H, H-arom), 9.50 (d, *J* = 3.6 Hz, 1 H, H-1) ppm. ¹³C NMR (75 MHz, CDCl₃): 6 = 34.4, 58.1, 127.1, 128.6, 128.8, 129.3, 129.3, 131.6, 133.4, 137.3, 194.2 ppm.

### Example 7. Preparation of 3-(Naphthalene-4-yl)-2-(phenylthio)propanal.

3-phenylprop-2-yn-1-ol (182 mg, 1 mmol), benzenethiol (154 µL, 1.5 mmol) and the catalyst NaAuCl₄·2H₂O (8 mg, 2 mol%) in 1,2-dichloroethane solvent (2.5 mL) were treated as described for 4-Phenyl-3-(phenylthio)butane-2-one to obtain the product as a reddish brown oil (257 mg, 0.88 mmol, 88% yield). IR (Neat): *v* = 3056, 1716, 1439, 776 cm⁻¹. ¹H NMR (300 MHz, CDCl₃): *δ* = 3.47 (dd, *J =* 6.0 Hz, 14.7 Hz, 1 H, H-3), 3.66 (dd, *J=* 8.1 Hz, 14.7 Hz, 1 H, H-3), 3.98-4.04 (m, 1 H, H-2), 7.29-7.33 (m, 3 H, H-arom), 7.38-7.46 (m, 4 H, H-arom), 7.50-7.60 (m, 2 H, H-arom), 7.80 (dd, *J =* 2.4 Hz, 7.2 Hz, 1 H, H-arom), 7.89-7.92 (m, 1 H, H-arom), 7.99 (dd, *J* = 1.2 Hz, 7.8 Hz, 1 H, H-arom), 9.54 (d, *J =* 3.6 Hz, 1 H, H-1) ppm. ¹³C NMR (75 MHz, CDCl₃): *δ* = 31.7, 57.3, 123.3, 125.6, 125.7, 126.1, 126.7, 127.9, 128.2, 128.7, 129.4, 131.7, 131.9, 133.4, 133.7, 134.3, 194.3 ppm. HRMS: calcd. for C₁₉H₁₆OSNa 315.0820; found 315.0813.

### Example 8. Preparation of 2-(4-Bromophenylthio)-3-phenylpropanal.

3-phenylprop-2-yn-1-ol (125 µL, 1 mmol), 4-bromobenzenethiol (284 mg, 1.5 mmol) and the catalyst NaAuCl₄·2H₂O (8 mg, 2 mol%) in 1,2-dichloroethane solvent (2.5 mL) were treated as described for 4-Phenyl-3-(phenylthio)butane-2-one to obtain the product as a yellowish oil (276 mg, 0.86 mmol, 86% yield). IR (Neat): *v* = 3421, 1716, 1471, 1007, 697 cm⁻¹. ¹H NMR (300 MHz, CDCl₃): 6 = 2.99 (dd, *J* = 6.9 Hz, 14.4 Hz, 1 H, H-3), 3.21 (dd, *J =* 8.1 Hz, 14.4 Hz, 1 H, H-3), 3.79-3.85 (m, 1 H, H-2), 7.21-7.38 (m, 7 H, H-arom), 7.42-7.45 (m, 2 H, H-arom), 9.48 (d, *J* = 3.6 Hz, 1 H, H-1) ppm. ¹³C NMR (100 MHz, CDCl₃): *δ*= 34.3, 57.9, 122.8, 127.0, 128.6, 129.0, 132.3, 134.5, 136.9, 137.7, 193.6 ppm. HRMS: calcd. for C₁₅H₁₃BrOSNa 342.9768; found 342.9746.

### Example 9. Preparation of 2-(4-Chlorophenylthio)-3-phenylpropanal.

3-phenylprop-2-yn-1-ol (125 µL, 1 mmol), 4-chlorobenzenethiol (218 mg, 1.5 mmol) and the catalyst NaAuCl₄·2H₂O (8 mg, 2 mol%) in 1,2-dichloroethane solvent (2.5 mL) were treated as described for 4-Phenyl-3-(phenylthio)butane-2-one to obtain the product as a yellowish oil (255 mg, 0.92 mmol, 92% yield). IR (Neat): *v* = 3415, 2923, 1717, 1474, 1092, 715 cm⁻¹. ¹H NMR (400 MHz, CDCl₃): *δ* = 2.98 (dd, *J* = 6.8 Hz, 14.4 Hz, 1 H, H-3), 3.21 (dd, *J =* 8.0 Hz, 14.4 Hz, 1 H, H-3), 3.77-3.82 (m, 1 H, H-2), 7.22-7.35 (m, 9 H, H-arom), 9.49 (d, *J=* 3.2 Hz, 1 H, H-1) ppm. ¹³C NMR (100 MHz, CDCl₃): *δ* = 34.3, 58.1, 127.0, 128.6, 129.0, 129.3, 130.1, 134.5, 134.7, 136.9, 193.6 ppm. HRMS: calcd. for C₁₅H₁₃ClOSNa 299.0273; found 299.0272.

### Example 10. Preparation of 2-(4-Fluorophenylthio)-3-phenylpropanal.

3-phenylprop-2-yn-1-ol (125 µL, 1 mmol), 4-fluorobenzenethiol (160 µL, 1.5 mmol) and the catalyst NaAuCl₄·2H₂O (8 mg, 2 mol%) in 1,2-dichloroethane solvent (2.5 mL) were treated as described for 4-Phenyl-3-(phenylthio)butane-2-one to obtain the product as a yellowish oil (125 mg, 0.48 mmol, 48% yield) at 90 °C for 48 h. IR (Neat): *v* = 3065, 1704, 1588, 1487, 1223, 826, 697 cm⁻¹.¹H NMR (400 MHz, CDCl₃): *δ* = 2.98 (dd, *J =* 6.8 Hz, 14.4 Hz, 1 H, H-3), 3.20 (dd, *J =* 8.0 Hz, 14.8 Hz, 1 H, H-3), 3.74-3.78 (m, 1 H, H-2), 6.99-7.04 (m, 2 H, H-arom), 7.22-7.40 (m, 7 H, H-arom), 9.51 (d, *J =* 3.6 Hz, 1 H, H-1) ppm. ¹³C NMR (100 MHz, CDCl₃): *δ* = 34.5, 58.8, 116.5, 116.7, 127.2, 128.9, 129.3, 136.4, 136.5, 137.4, 164.6, 193.9 ppm. HRMS: calcd. for C₁₅H₁₃FOSNa 283.0569; found 283.0582.

### Example 11. Preparation of 2-(4-Isopropylphenylthio)-3-phenylpropanal.

3-phenylprop-2-yn-1-ol (125 µL, 1 mmol), 4-isopropylbenzenethiol (233 µL, 1.5 mmol) and the catalyst NaAuCl₄·2H₂O (8 mg, 2 mol%) in 1,2-dichloroethane solvent (2.5 mL) were treated as described for 4-Phenyl-3-(phenylthio)butane-2-one to obtain the product as a yellowish oil (125 mg, 0.48 mmol, 48% yield) (216 mg, 0.76 mmol, 76% yield). IR (Neat): *v* = 2960, 1719, 825 cm⁻¹. ¹H NMR (400 MHz, CDCl₃): *δ* = 1.27 (d, *J=* 6.8 Hz, 6 H, H-methyl), 2.88-2.95 (m, 1 H, C*H*(CH₃)₂), 3.01 (dd, *J =* 6.8 Hz, 14.8 Hz, 1 H, H-3), 3.23 (dd, *J =* 7.6 Hz, 14.4 Hz, 1 H, H-3), 3.78-3.82 (m, 1 H, H-2), 7.19 (d, *J* = 8.0 Hz, 2 H, H-arom), 7.25-7.29 (at, 3 H, H-arom), 7.32-7.35 (at, 4 H, H-arom), 9.53 (d, *J=* 3.6 Hz, 1 H, H-1) ppm. ¹³C NMR (100 MHz, CDCl₃): *δ* = 24.0, 34.0, 34.6, 58.6, 127.1, 127.6, 128.4, 128.8, 129.4, 134.0, 137.7, 149.8, 194.3 ppm. HRMS: calcd. for C₁₈H₂₀OSNa 307.1133; found 307.1164.

### Example 12. Preparation of 2-(4-methoxyphenylthio)-3-phenylpropanal.

3-phenylprop-2-yn-1-ol (125 µL, 1 mmol), 4-methoxybenzenethiol (184 µL, 1.5 mmol) and the catalyst NaAuCl₄·2H₂O (8 mg, 2 mol%) in 1,2-dichloroethane solvent (2.5 mL) were treated as described for 4-Phenyl-3-(phenylthio)butane-2-one to obtain the product as reddish yellow oil (117 mg, 0.43 mmol, 43% yield). IR (Neat): *v* = 2836, 1717, 1590, 1490, 1248, 824, 698 cm⁻¹. ¹H NMR (400 MHz, CDCl₃): *δ* = 2.96 (dd, *J=* 6.8 Hz, 14.4 Hz, 1 H, H-3), 3.16 (dd, *J =* 8 Hz, 14.4 Hz, 1 H, H-3), 3.68-3.79 (m, 1 H, H-2), 3.82 (s, 3 H, H-methoxy), 6.85-6.88 (m, 2 H, H-arom), 7.25-7.28 (m, 2 H, H-arom), 7.32-7.35 (m, 3 H, H-arom), 7.36-7.44 (m, 2 H, H-arom), 9.51 (d, *J=* 3.2 Hz, 1 H, H-1) ppm. ¹³C NMR (100 MHz, CDCl₃): *δ* = 33.9, 55.3, 58.7, 114.6, 114.7, 126.8, 128.6, 129.0, 132.6, 136.7, 193.7 ppm. HRMS: calcd. for C₁₆H₁₆O₂SNa 295.0769; found 295.0770.

### Example 13. Preparation of 4-Phenyl-3-(phenylthio)butane-2-one.

At first, the catalyst NaAuCl₄·2H₂O (8 mg, 2 mol%) was weighed and transferred to a 5 mL microwave vial containing a small magnet in a glove box under nitrogen atmosphere. After that, the cap of the vial was closed tightly and the vial was taken out from the glove-box. 2.5 mL of dry 1,2-dichloroethane solvent followed by 4-phenyl-3-butyn-2-ol (145 µL, 1 mmol) and benzenethiol (154 µL, 1.5 mmol) were added to the vial by syringe and was stirred using a magnetic stirrer at 65 °C for 24 h. After completion of the reaction (by TLC), 1,2 dichloroethane was evaporated under reduced pressure and the residue was purified by silica-gel (100-200 mess) column chromatography using 3% (*v*/*v)* ethyl acetate / pentane solution to afford the desired product as a pale yellow oil (233 mg, 0.91 mmol, 91% yield). ¹H NMR (300 MHz, CDCl₃): *δ* = 2.20 (s, 3 H, H-1), 3.00 (dd, *J =* 6.9 Hz, 14.4 Hz, 1 H, H-4), 2.68 (dd, *J =* 8.4 Hz, 14.1 Hz, 1 H, H-4), 3.90 (dd, *J* = 6.9 Hz, 8.4 Hz, 1 H, H-3), 7.18-7.37 (m, 10 H, H-arom) ppm. ¹³C NMR (75 MHz, CDCl₃): *δ* = 28.1, 36.9, 59.0, 127.1, 128.5, 128.8, 129.4, 133.0, 133.3, 138.3, 204.5 ppm.

### Example 14. Preparation of 6-phenyl-3-(phenylthio)hexan-2-one.

At first, the catalyst CuI (4 mg, 2 mol%) was weighed and transferred to a 5 mL microwave vial containing a small magnet under nitrogen atmosphere. After that, the cap of the vial was closed tightly. 2.0 mL of dry 1,2-dichloroethane (DCE) solvent followed by 6-phenylhex-3-yn-2-ol (174 mg dissolved in 0.5 mL DCE, 1 mmol) and benzenethiol (205 µL, 2 mmol) were added to the vial by syringe and was stirred using a magnetic stirrer at 90 °C for 24 h. After completion of the reaction (by TLC), 1,2 dichloroethane was evaporated under reduced pressure and the residue was purified by silica-gel (100-200 mess) column chromatography using 3% (*v*/*v*) ethyl acetate / pentane solution to afford the desired product as a pale yellow oil (187 mg, 0.66 mmol, 66% yield). ¹H NMR (300 MHz, CDCl₃): *δ* = 1.73-1.89 (m, 4 H, H-4 & H-5), 2.24 (s, 3 H, H-1), 2.67 (t, *J =* 8.0 Hz, 2 H, H-6), 3.64 (t, *J=* 8.0 Hz, 1 H, H-3), 7.17-7.37 (m, 10 H, H-aromatic) ppm. ¹³C NMR (100 MHz, CDCl₃): *δ* = 26.5, 28.9, 29.7, 35.4, 57.6, 125.9, 127.8, 128.3, 129.0, 132.4, 132.8, 141.5, 205.0 ppm.

### Example 15. Preparation of 3-phenyl-2-(phenylthio)propanal.

At first, the catalyst CuI (4 mg, 2 mol%) was weighed and transferred to a 5 mL microwave vial containing a small magnet under nitrogen atmosphere. After that, the cap of the vial was closed tightly. 2.5 mL of distilled water followed by 3-phenylprop-2-yn-1-ol (125 µL, 1 mmol) and benzenethiol (205 µL, 2 mmol) were added to the vial by syringe and was stirred using a magnetic stirrer at 110 °C for 48 h. After completion of the reaction (by TLC), 1,2 dichloroethane was evaporated under reduced pressure and the residue was purified by silica-gel (100-200 mess) column chromatography using 3% (*v*/*v*) ethyl acetate / pentane solution to afford the desired product as a pale yellow oil (225 mg, 0.93 mmol, 93% yield). ¹H NMR (300 MHz, CDCl₃): *δ* = 3.00 (dd, *J =* 6.9 Hz, 14.4 Hz, 1 H, H-3), 3.22 (dd, *J* = 8.1 Hz, 14.4 Hz, 1 H, H-3), 3.81-3.87 (m, 1 H, H-2), 7.23-7.41 (m, 10 H, H-arom), 9.50 (d, *J* = 3.6 Hz, 1 H, H-1) ppm. ¹³C NMR (75 MHz, CDCl₃): *δ* = 34.4, 58.1, 127.1, 128.6, 128.8, 129.3, 129.3, 131.6, 133.4, 137.3, 194.2 ppm.

### Example 16. Preparation of 3-(benzylthio)butan-2-one.

At first, the catalyst AuCl (12 mg, 5 mol%) and Pd(OAc)₂ (11 mg, 5 mol%) was weighed and transferred to a 5 mL microwave vial containing a small magnet under nitrogen atmosphere. After that, the cap of the vial was closed tightly. 2.5 mL of dry nitromethane solvent followed by but-3-yn-2-ol (78 µL, 1 mmol) and phenylmethanethiol (153 µL, 1.3 mmol) were added to the vial by syringe and was stirred using a magnetic stirrer at 100 °C for 24 h. After completion of the reaction (by TLC), nitromethane was evaporated under reduced pressure and the residue was purified by silica-gel (100-200 mess) column chromatography using 3% (*v*/*v*) ethyl acetate / pentane solution to afford the desired product as a pale yellow oil (176 mg, 0.91 mmol, 91% yield). ¹H NMR (300 MHz, CDCl₃): *δ* = 1.38 (d, *J* = 8 Hz, 3 H, H-methyl), 2.24 (s, 3 H, H-1), 3.22-3.38 (m, 1 H, H-3), 3.60-3.78 (m, 2 H, H-4), 7.25-7.34 (m, 5 H, H-arom) ppm. ¹³C NMR (75 MHz, CDCl₃): *δ* = 16.2, 35.3, 43.6, 48.6, 128.7, 128.7, 129.3, 129.6, 204.2 ppm.

## Claims

1. A method of preparing an α-sulfenylated carbonyl compound according to the present invention comprising:
- providing reactants comprising a primary or a secondary propargylic alcohol, a thiol and a transition metal catalyst wherein the catalyst is a catalyst comprising gold(III), gold(I), copper(I), palladium(II) or platinum(II) or mixtures thereof such as gold and palladium, or copper and palladium, or platinum and palladium;
- providing a solvent;
- mixing the reactants and the solvent;
- letting the reactants react.

2. The method according to claim 1 wherein the method is performed in one step with no intermediate steps.

3. The method according to any one of claims 1 and 2 conducted in an inert atmosphere or in an open atmosphere.

4. The method according to any one of the preceding claims wherein the thiol is an aryl or alkyl thiol.

5. The method according to any one of the preceding claims wherein the yield of the compound is 55 to 100%, preferably 80 to 100%.

6. The method according to any one of the preceding claims wherein the catalyst is a gold(I) chloride or copper(I) iodide catalyst.

7. The method according to claim 1 wherein the method is performed in one step with no intermediate steps and wherein the thiol is a benzene thiol and the catalyst is a gold(I) chloride catalyst.

8. The method according to anyone of claims 1 to 3 wherein the transition metal catalyst is a mixture of gold(I) and palladium(II) and the thiol is an alkylthiol.

9. Use of a primary or a secondary propargylic alcohol and a thiol, a transition metal catalyst wherein the catalyst is a catalyst comprising gold(III), gold(I), copper(I), palladium(II), platinum(II) or mixtures thereof such as gold and palladium, or copper and palladium, or platinum and palladium and a solvent for preparing an α-sulfenylated carbonyl compound.

## Patentansprüche

1. Ein Verfahren zur Herstellung einer α-sulfenylierten Carbonylverbindung gemäß der vorliegenden Erfindung, umfassend:
- Bereitstellen von Reaktanten, umfassend einen primären oder einen sekundären Propargylalkohol, ein Thiol und einen Übergangsmetallkatalysator, wobei der Katalysator ein Katalysator ist, welcher Gold(III), Gold(I), Kupfer(I), Palladium(II) oder Platin(II) oder Gemische davon, wie Gold und Palladium, oder Kupfer und Palladium, oder Platin und Palladium, umfasst;
- Bereitstellen eines Lösungsmittels;
- Mischen der Reaktanten und des Lösungsmittels;
- Reagieren lassen der Reaktanten.

2. Das Verfahren gemäß Anspruch 1, wobei das Verfahren in einem Schritt ohne Zwischenschritte durchgeführt wird.

3. Das Verfahren gemäß einem der Ansprüche 1 und 2, das in einer inerten Atmosphäre oder in einer offen Atmosphäre durchgeführt wird.

4. Das Verfahren gemäß einem der vorstehenden Ansprüche, wobei das Thiol ein Aryl- oder Alkylthiol ist.

5. Das Verfahren gemäß einem der vorstehenden Ansprüche, wobei die Ausbeute der Verbindung 55 bis 100 %, vorzugsweise 80 bis 100 %, beträgt.

6. Das Verfahren gemäß einem der vorstehenden Ansprüche, wobei der Katalysator ein Gold(I)chlorid- oder Kupfer(I)iodid-Katalysator ist.

7. Das Verfahren gemäß Anspruch 1, wobei das Verfahren in einem Schritt ohne Zwischenschritte durchgeführt wird und wobei das Thiol ein Benzolthiol ist und der Katalysator ein Gold(I)chlorid-Katalysator ist.

8. Das Verfahren gemäß einem der Ansprüche 1 bis 3, wobei der Übergangsmetallkatalysator ein Gemisch aus Gold(I) und Palladium(II) ist und das Thiol ein Alkylthiol ist.

9. Verwendung eines primären oder eines sekundären Propargylalkohols und eines Thiols, eines Übergangsmetallkatalysators, wobei der Katalysator ein Katalysator ist, welcher Gold(III), Gold(I), Kupfer(I), Palladium(II), Platin(II) oder Gemische davon, wie Gold und Palladium, oder Kupfer und Palladium, oder Platin und Palladium umfasst, und eines Lösungsmittels zur Herstellung einer α-sulfenylierten Carbonylverbindung.

## Revendications

1. Procédé de préparation d'un composé α-sulfénylé carbonyle selon la présente invention, comprenant :
- la fourniture de réactifs comprenant un alcool propargylique primaire ou secondaire, un thiol et un catalyseur à base de métal de transition dans lequel le catalyseur est un catalyseur comprenant de l'or(III), de l'or(I), du cuivre(I), du palladium(II) ou du platine(II) ou des mélanges de ceux-ci tels que d'or et de palladium, ou de cuivre et de palladium, ou de platine et de palladium ;
- la fourniture d'un solvant ;
- le mélange des réactifs et du solvant ;
- le fait de laisser les réactifs réagir.

2. Procédé selon la revendication 1, dans lequel le procédé est réalisé en une étape sans étape intermédiaire.

3. Procédé selon l'une quelconque des revendications 1 et 2, réalisé dans une atmosphère inerte ou dans une atmosphère ouverte.

4. Procédé selon l'une quelconque des revendications précédentes, dans lequel le thiol est un aryl ou alkyl thiol.

5. Procédé selon l'une quelconque des revendications précédentes, dans lequel le rendement du composé est de 55 à 100 %, de préférence de 80 à 100 %.

6. Procédé selon l'une quelconque des revendications précédentes, dans lequel le catalyseur est un catalyseur de chlorure d'or(I) ou d'iodure de cuivre(I).

7. Procédé selon la revendication 1, dans lequel le procédé est réalisé en une étape sans étape intermédiaire et dans lequel le thiol est un benzène thiol et le catalyseur est un catalyseur de chlorure d'or(I).

8. Procédé selon l'une quelconque des revendications 1 à 3, dans lequel le catalyseur à base de métal de transition est un mélange d'or(I) et de palladium(II) et le thiol est un alkylthiol.

9. Utilisation d'un alcool propargylique primaire ou secondaire et d'un thiol, d'un catalyseur à base de métal de transition dans lequel le catalyseur est un catalyseur comprenant de l'or(III), de l'or(I), du cuivre(I), du palladium(II), du platine(II) ou des mélanges de ceux-ci tels que d'or et de palladium, ou de cuivre et de palladium, ou de platine et de palladium et d'un solvant pour la préparation d'un composé α-sulfénylé carbonyle.
